# EUROPEAN PATENT APPLICATION

(11) **EP 1 675 121 A1**
(43) Date of publication of application: **28.06.2006**
(21) Application number: 05110814.0
(22) Date of filing: 16.11.2005
(51) Int. Cl.: G11B 20/12, G11B 27/10, G11B 27/30, G11B 27/034

(54) **Information recording medium, information reproducing apparatus, and information reproducing method**

(30) Priority: 24.12.2004 JP 2004373904
(71) Applicant: KABUSHIKI KAISHA TOSHIBA, Tokyo 105-8001 (JP)
(72) Inventor: Suzuki, Nobuyuki Toshiba Corporation I.P.D., 105-8001 Tokyo (JP)
(74) Representative: Henkel, Feiler & Hänzel

(57) **Abstract**

A region in which at least one of subsidiary video data (11) and subsidiary audio data (12) packed after identification information (stream_id) has been assigned thereto is provided in a region in which a main stream of an information recording medium (100) is to be recorded. The main stream is an MPEG2 program stream, and at least one of the video data and the audio data is packed as a packet identified by private_stream_2.

## Description

The present invention relates to an information recording medium such as an optical disk, for example. In addition, the present invention relates to an information reproducing apparatus and an information reproducing method for carrying out reproduction of information from the information recording medium.

As is well known, for example, in a recording and reproducing system using an optical disk such as a digital versatile disk (DVD), there has been devised adding a comment by using subsidiary video data or subsidiary audio data to a video image or a voice of a main stream, i.e., mounting a so-called commentary video or commentary audio function.

In the meantime, in achieving such a commentary video or commentary audio function, when downloading subsidiary video data or subsidiary audio data from an optical disk, it is necessary to take consideration so as not to preclude reproduction of the video image or voice of the main stream.

In Jpn. Pat. Appln. KOKAI Publication No. 2002-247526, there is disclosed a configuration for reproducing externally captured audio stream data or sub-picture stream data in synchronism with video stream data read from a DVD video disk according to a user operation.

The present invention has been made in view of the above described circumstance. It is an object of the present invention to provide an information recording medium, an information reproducing apparatus, and an information reproducing method which enable acquisition of subsidiary video data or subsidiary audio data while reproducing a main stream.

According to one aspect of the present invention, there is provided an information recording medium configured to have, in a region in which a main stream is recorded, a region in which at least one of subsidiary video data and subsidiary audio data packed after identification information has been assigned thereto is recorded.

According to one aspect of the present invention, there is provided an information reproducing apparatus for reproducing an information recording medium having a region in which at least one of subsidiary video data and subsidiary audio data packed after identification information has been assigned thereto is recorded in a region in which a main stream is recorded, the apparatus comprising: a reproducing section configured to read the main stream from the information recording medium and to apply a decode processing operation to at least one of video data and audio data packed after identification information included in the main stream has been assigned thereto; and a storage section configured to store at least one of the subsidiary video data and subsidiary audio data packed after identification information included in the main stream read from the information recording medium has been assigned thereto during a decode processing operation using the reproducing section.

According to one aspect of the present invention, there is provided an information reproducing method for reproducing an information recording medium which has a region in which at least one of subsidiary video data and subsidiary audio data packed after identification information has been assigned thereto is recorded in a region in which a main stream is recorded, the method comprising: a reproducing step of applying a decode processing operation to at least one of video data and audio data packed after reading the main stream from the information recording medium and identification information included in the main stream has been assigned thereto; and a storing step of storing at least one of the subsidiary video data and subsidiary audio data packed after identification information included in the main stream read from the information recording medium has been assigned thereto during a decode processing operation by the reproducing step.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a view showing an embodiment of the present invention, the view being adopted to explain a stream structure of information recorded in an optical disk;
FIG. 2 is a view adopted to explain a subsidiary video pack contained in the information recorded in the optical disk in accordance with the embodiment;
FIG. 3 is a view adopted to explain a full detail of a video pack header contained in the subsidiary video pack in accordance with the embodiment;
FIG. 4 is a view adopted to explain a full detail of a subsidiary video packet contained in the subsidiary video pack in accordance with the embodiment;
FIG. 5 is a view adopted to explain a subsidiary audio pack contained in the information recorded in the optical disk in accordance with the embodiment;
FIG. 6 is a view adopted to explain a full detail of a subsidiary audio packet contained in the subsidiary audio pack in accordance with the embodiment;
FIG. 7 is a block diagram adopted to explain an optical disk device for reproducing an optical disk in accordance with the embodiment;
FIG. 8 is a view adopted to schematically explain a main operation of the optical disk device in accordance with the embodiment;
FIG. 9 is a view adopted to explain a stream structure of information recorded in an optical disk in accordance with a DVD-Video standard;
FIG. 10 is a view adopted to explain a navigation pack, a video pack, an audio pack, and a subsidiary picture pack in accordance with the DVD-Video standard, respectively;
FIG. 11 is a view adopted to explain a detailed structure of a pack header in accordance with the DVD-Video standard;
FIG. 12 is a view adopted to explain a detailed structure of a packet in accordance with the DVD-Video standard;
FIG. 13 is a view adopted to explain a full detail of stream_id and sub stream_id in accordance with the DVD-Video standard; and
FIG. 14 is a view adopted to explain a data structure of management information in accordance with the DVD-Video standard.

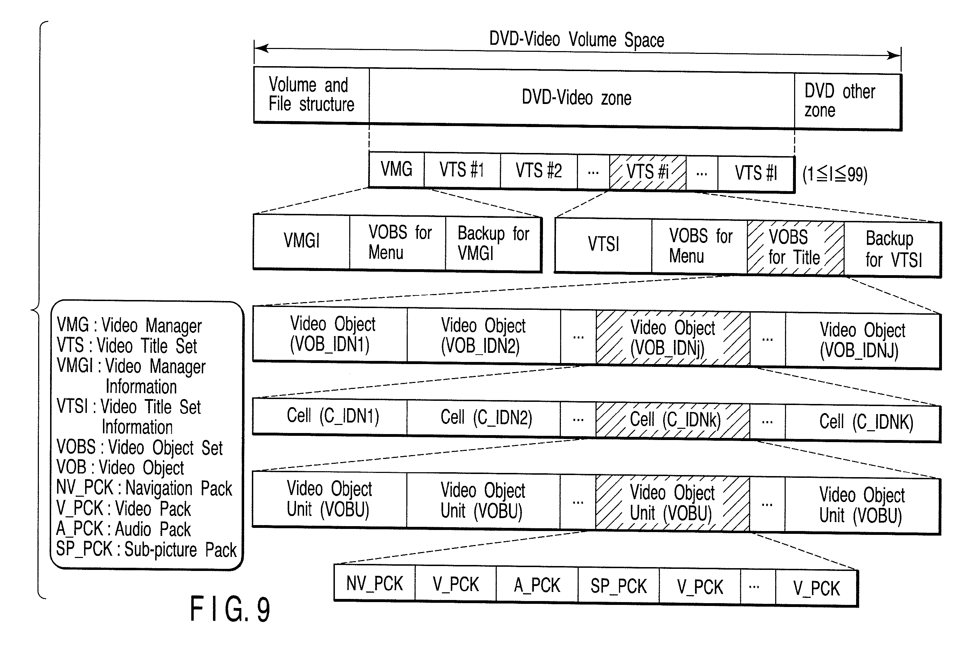

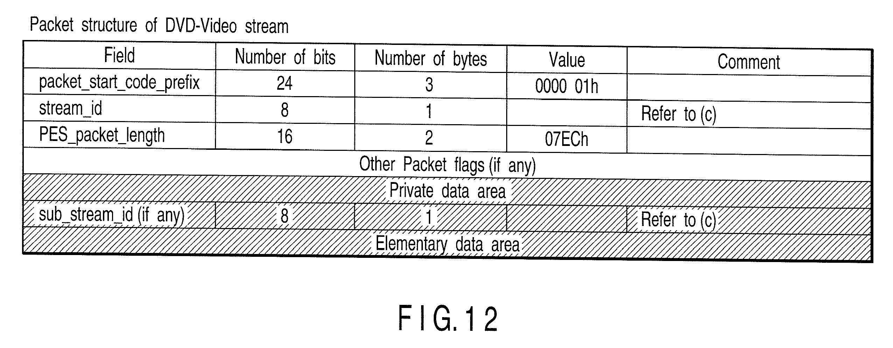

Hereinafter, an embodiment of the present invention will be described in detail with reference to the accompanying drawings. First, a data structure of a DVD-Video standard will be described with reference to FIGS. 9 to 14.

FIG. 9 shows a stream structure in accordance with the DVD-Video standard. A volume space is composed of: a volume and file configuration zone, a DVD video zone, and another zone. A universal disk format (UDF) bridge configuration is described in the volume and file configuration zone so that the data can be read by a computer which conforms to a predetermined standard.

The DVD video zone has a video manager (VMG) and a video title set (VTS). The video manager (VMG) and the video title set (VTS) each are composed of a plurality of files. The video manager (VMG) is provided as information for controlling the video title set (VTS).

The video manager (VMG) has: video manager information (VMGI) serving as control data for controlling a video title set (VTS) or the like; a video object set (VMGM_VOBS) serving as data for menu display; and video manager information (VMGI) for backup.

The video title set (VTS) has: video title set information (VTSI) serving as control data; a video object set (VMGM_VOBS) serving as data for menu display; a video object set (VTSTT_VOBS) for a title of a video title set which is a video object set for video image display; and video title set information (VTSI) for backup.

Further, a video object set (VTSTT_VOBS) for video image display is composed of a plurality of cells. A cell ID number is assigned to each cell (Cell).

FIG. 9 hierarchically shows a relationship between the above described video object set (VOBS) and a cell (Cell) and the contents of the cell (Cell). When a DVD reproducing process is carried out, a video image transition (such as scene change, angle change, and story change) or special reproduction is handled in units of cells (Cell) or in units of video object units (VOBU) which is a lower layer of the cell units.

The video object set (VOBS) is composed of one or a plurality of video objects (VOB_IDN1 to VOB_IDNi). Further, one video object is composed of one or a plurality of cells (C_IDN1 to C_IDNj).

In addition, one cell (Cell) is composed of one or a plurality of video object units (VOBU). In addition, one video object unit (VOBU) is composed of: one navigation pack (NV_PCK); one or a plurality of video packs (V_PCK); one or a plurality of audio packs (A_PCK); and one or a plurality of subsidiary picture packs (SP_PCK).

The navigation pack (NV_PCK) is mainly used as control data for making reproduction display control of data contained in a video object unit (VOBU) to which the navigation pack belongs; and control data for making a data search for the video object unit (VOBU).

The video pack (V PCK) is provided as main video image information and is compressed in accordance with MPEG (moving picture experts group) 2 standard. In addition, the subsidiary picture pack (SP_PCK) is provided subsidiary picture information having subsidiary contents with respect to the main video image. For example, this information is cinema subtitles or scenario, and a run length compression technique is used. The audio pack (A_PCK) is provided as voice information.

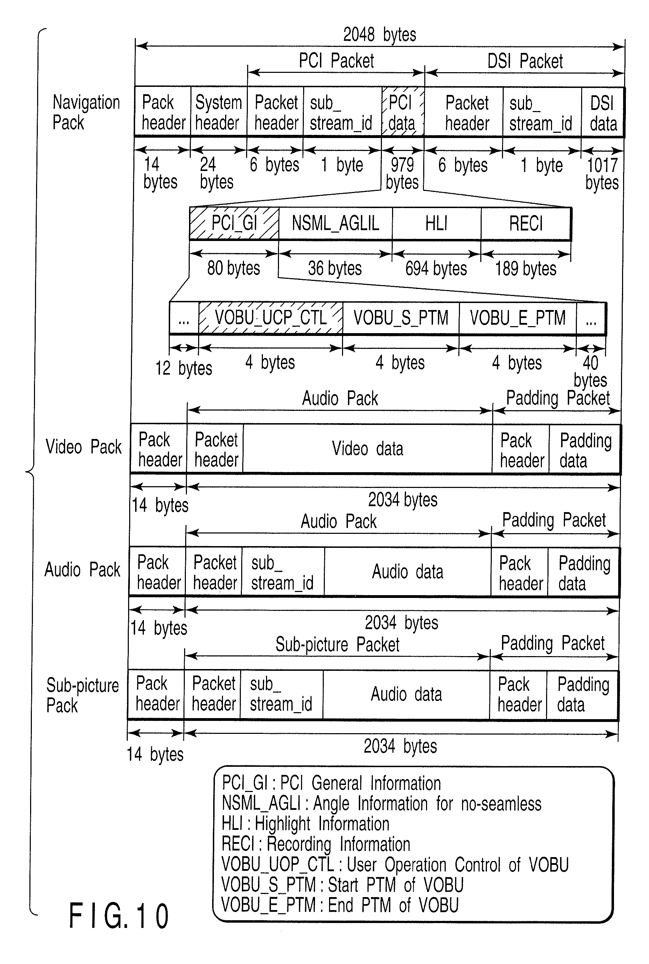

FIG. 10 shows a structure of a navigation pack (NV_PCK), a video pack (V_PCK), an audio pack (A_PCK), and a subsidiary picture pack (SP_PCK) in accordance with a DVD-Video standard.

The navigation pack (NV_PCK) is composed of two packets, i.e., a pack header and a system header. That is, this pack is a PCI packet (PCI_PKT) and a DSI packet (DSI_PKT) .

The PCI packet (PCI_PKT) consists of a packet header and a private data area. sub_stream_id exists in a first byte of the private data area followed by PCI data for making reproduction control of a video object unit (VOBU). This PCI data includes PCI general information (PCI_GI), non-seamless angle information (NSML_AGLI), highlight information (HLI), and recording information (RECI).

The PCI general information (PCI_GI) includes a VOBU user operation control (VOBU_UOP_CTL) and describes user information disabled for each video object unit (VOBU). In addition, the PCI general information describes a picture display start time (VOBU_S_PTM) first displayed in the video object unit (VOBU); and a picture display end time (VOBUI_E_PTM) or the like lastly displayed in the video object unit (VOBU).

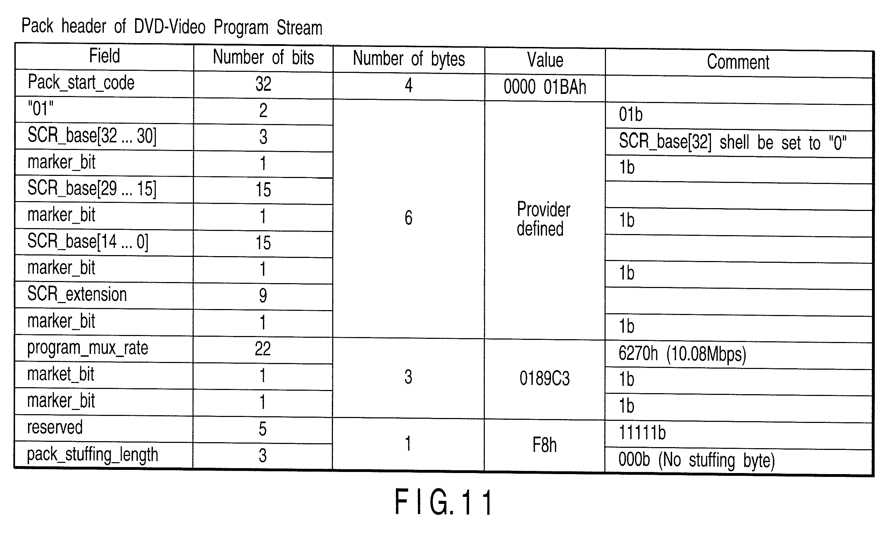

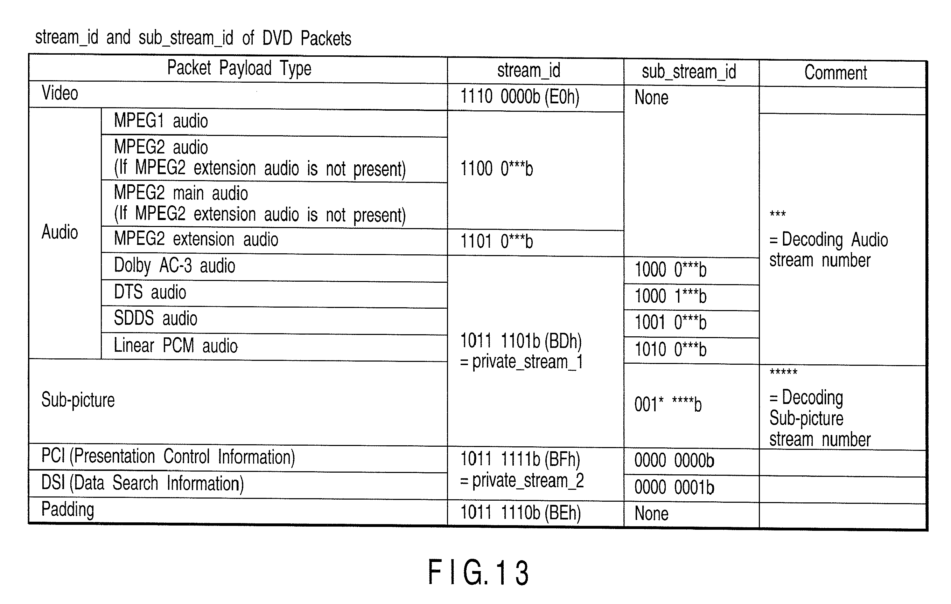

FIGS. 11 to 13 each show a pack header and a packet internal flag in accordance with a DVD-Video standard. In particular, FIG. 13 shows stream_id and sub_stream_id of a packet which includes each elementary stream. A DVD player carries out separation of the elementary streams by using these stream_id and sub_stream_id.

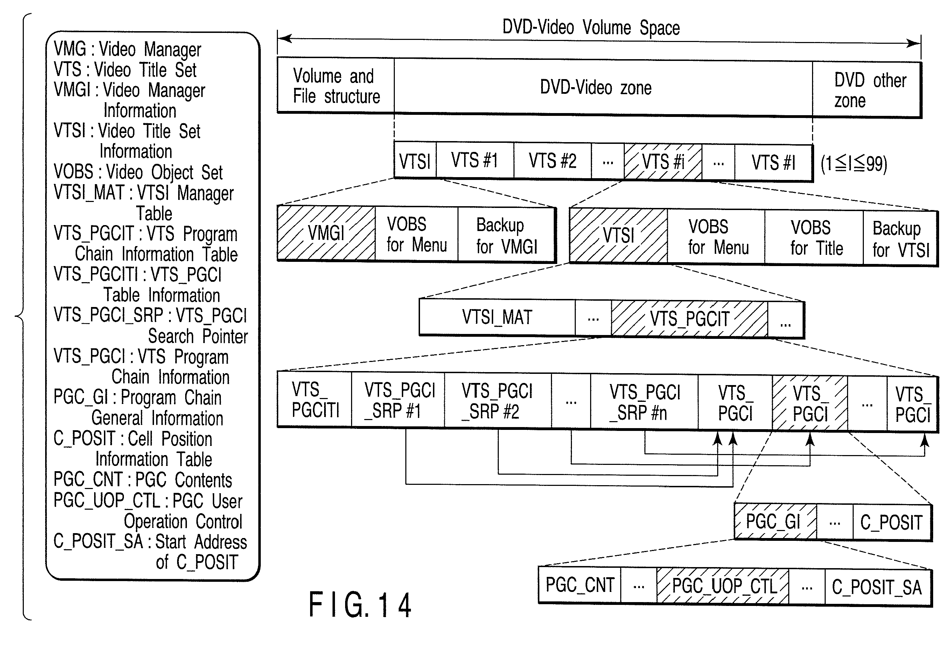

FIG. 14 shows the contents of management information in accordance with the DVD-Video standard. A video title set program chain information table (VTS_PGCIT) is described in video title set information (VTSI). Therefore, when a video object set (VOBS) in one video title set (VTS) is reproduced, program chain selected by a user from among a plurality of program chains presented in this video title set program chain information table (VTS_PGCIT) is utilized.

This VTS program chain information table (VTS_PGCIT) describes information (VTS_PGCI) relating to a VTS program chain (VTS_PGC). In addition, this table describes: information (VTS_PGCITI) on an information table (VTS_PGCIT) relating to a VTS program chain (VTS_PGC); a VTS_PGCI search pointer (VTS_PGCI_SRP) for making a search for a VTS program chain (VTS_PGC) by the number (from #1 to #n) of VTS program chains (VTS_PGC); and information (VTS_PGCI) relating to each VTS program chain (VTS_PGC) by the number (from #1 to #n) corresponding to such VTS program chains (VTS_PGC).

PGC information (VTS_PGCI) contained in a video title set (VTS) describes: program chain general information (PGC_GI); a program chain command table (PC_CMDT); a program chain program map (PGC_PGMAP); a cell reproduction information table (C_PBIT); and a cell position information table (C_POSIT).

The program chain general information (PGC_GI) includes a PGC user operation control (PGC_UOP_CTL) and describes a user operation disabled for each program chain (PGC).

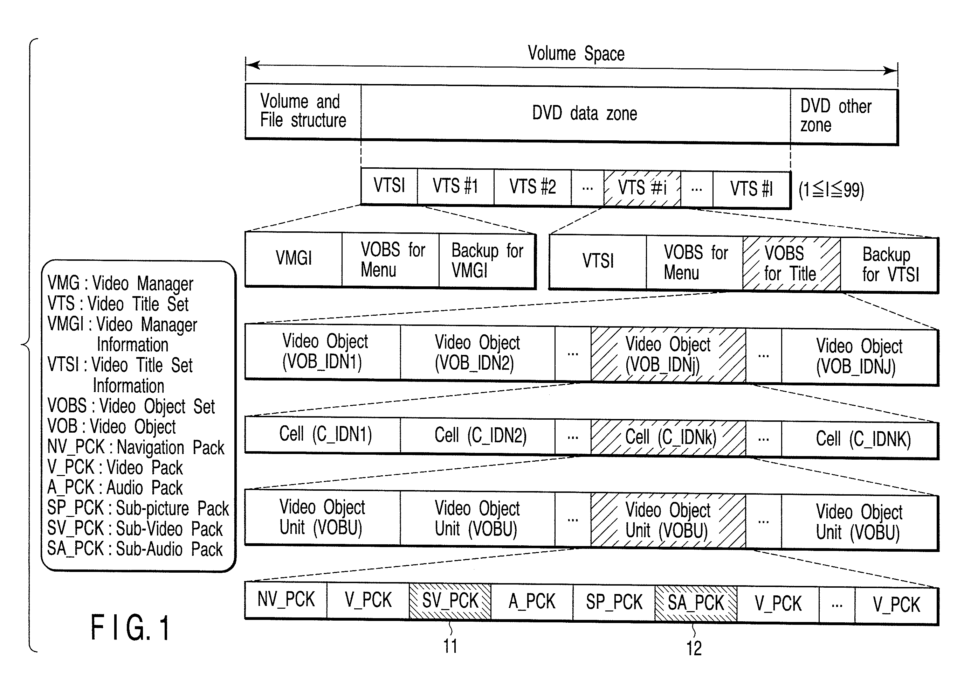

FIG. 1 shows a stream structure in accordance with the present embodiment. As compared with a stream structure shown in FIG. 9, a subsidiary video pack (PV_PCK) 11 and a subsidiary audio pack (SA_PCK) 12 are newly provided in a video object unit (VOBU).

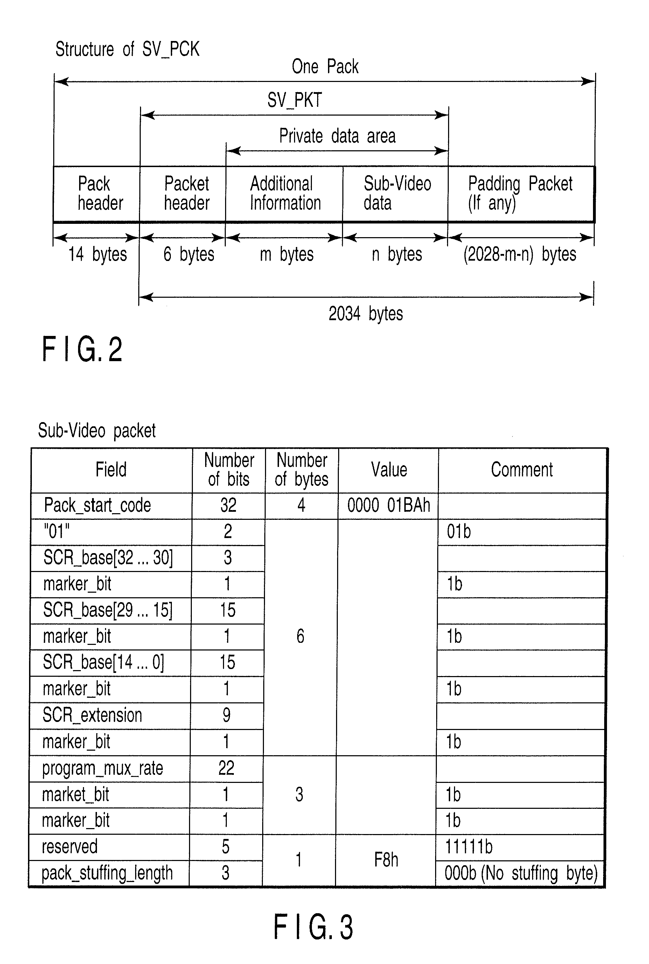

FIG. 2 shows a structure of the subsidiary video pack (SV_PCK) 11. The subsidiary video pack (SV_PCK) 11 is composed of a pack header and one subsidiary video packet (SV_PKT), and may include one padding packet for pack length adjustment as required.

FIG. 3 shows the contents of a pack header of the subsidiary video pack (SV_PCK) 11. This pack header describes a time SCR at which this pack arrives at an input buffer of each decoder.

On the other hand, the subsidiary video packet (SV_PKT) is composed of a packet header and a private data region.

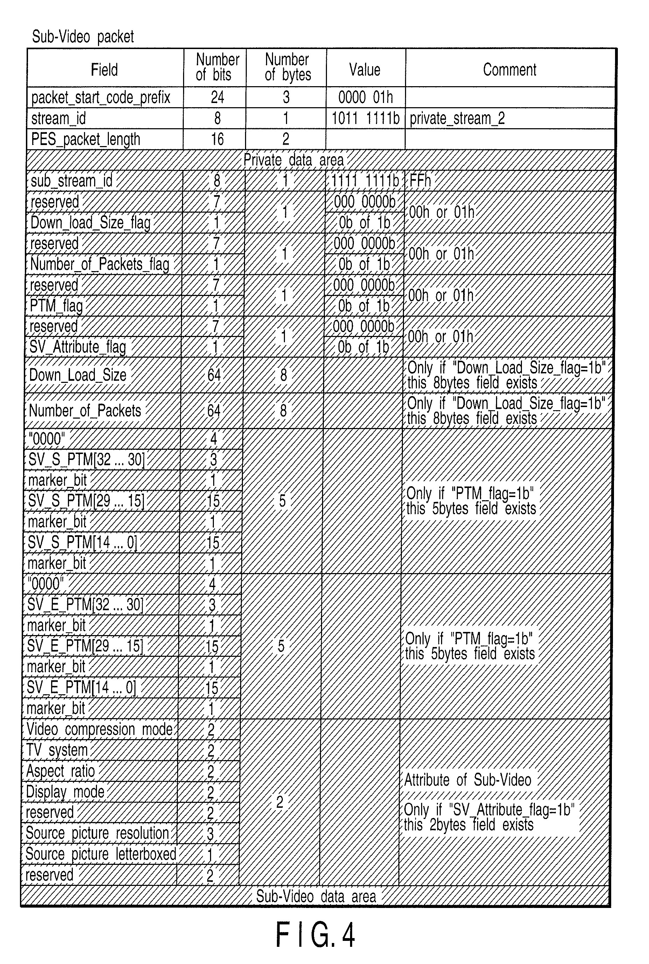

FIG. 4 shows the contents of a subsidiary video packet (SV_PKT). A packet header section describes stream id = BFh which is its identification information and indicates a format of private_stream_2. In this manner, subsidiary video data is not subject to a limitation that a data retention period in an input buffer of a decoder is equal to or shorter than 1 second, the limitation being defined in an MPEG2 program stream.

A private data region is divided into an additional information (additional_information) region and a subsidiary video data region. The additional information region describes sub_stream_id = FFh followed by: a download size presence flag (down_load_size_flag); a packet number presence flag (number_of_packets_flag); a PTM presence flag (PTM_flag); and a subsidiary video attribute presence flag (SV_attribute_flag). When down_load_size_flag = 1, a download size (down_load_size) exists. When number_of_packets_flag = 1, the number of download packets (number_of_packets) exists. When PTM_flag = 1, a subsidiary video display start time (SV_S_PTM) and a subsidiary video display end time (SV_E_PTM) exist. When SV_attribute_flag = 1, subsidiary video attribute information (SV_attribute) exists. In the case where these flags are 0, a corresponding region does not exist.

The download size (down load size) describes size of all subsidiary video data to be downloaded. The number of download packets (number_of_packets) describes a packet number of data for transferring all of these items of subsidiary video data. The subsidiary video display start time (SV_S_PTM) describes a display start time of subsidiary picture data in units of 90 kHz with precision of 33 bits. The subsidiary video display end time (SV_E_PTM) describes a display end time of subsidiary picture data in units of 90 kHz with precision of 33 bits. The subsidiary video attribute information (SV_attribute) includes: a video encoding scheme (video-compression_mode); a television system (TV_system); an aspect ratio (aspect_ratio); a display mode (display_mode); a source picture resolution (source_picture_resolution); and source picture letterbox information (source_picture_letterboxed) or the like.

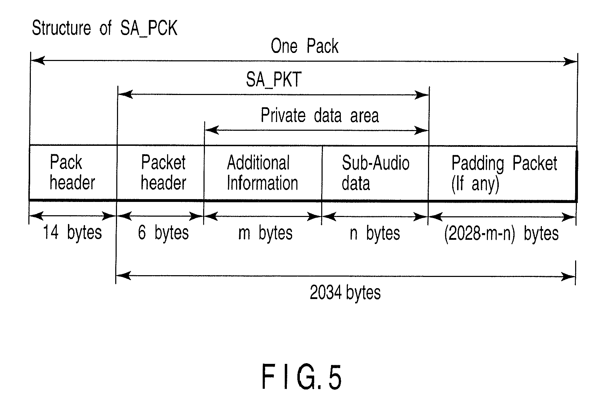

FIG. 5 shows a structure of the above described subsidiary audio pack (SA_PCK) 12. The subsidiary audio pack (SA_PCK) 12 is composed of a pack header and one subsidiary audio packet (SA_PKT). This pack may include one padding packet for pack length adjustment as required. The contents of a pack header of the subsidiary audio pack (SA_PCK) 12 are identical to those of a pack header of the subsidiary video pack (SV_PCK) 11 shown in FIG. 3.

The subsidiary audio packet (SA_PKT) is composed of a packet header and a private data region.

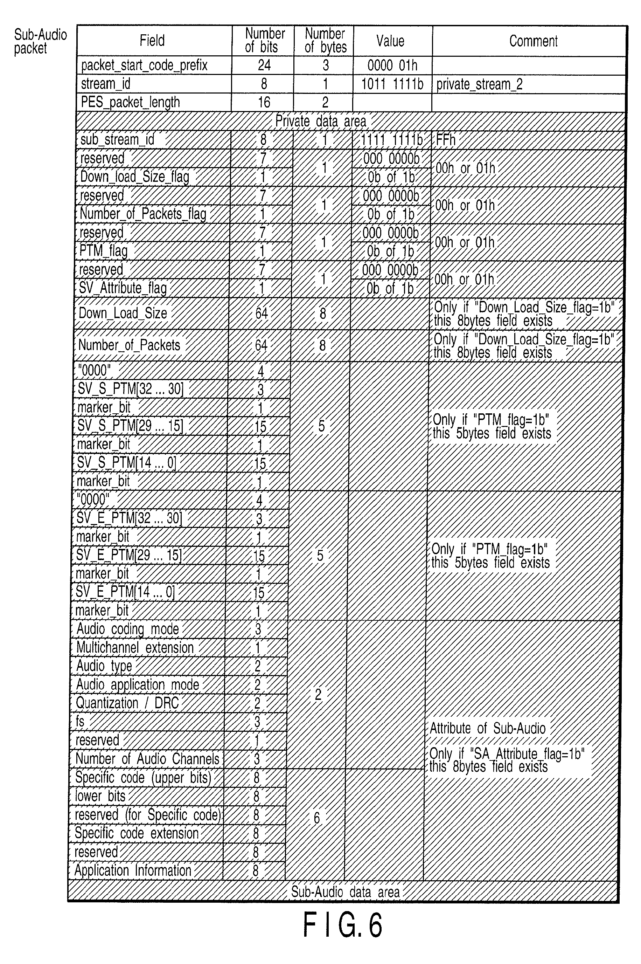

FIG. 6 shows the contents of a subsidiary audio packet (SA_PKT). A packet header section describes stream_id = BFh which is its identification information and indicates a format of private_stream_2. In this manner, the subsidiary audio data is not subject to a limitation that a data retention period in an input buffer of a decoder is equal to or shorter than 1 second, the limitation being defined in an MPEG2 program stream.

The private data region is divided into an additional information (additional_information) region and a subsidiary audio data region. The additional information region describes sub-stream_id = FEh followed by a download size presence flag (down_load_size_flag), a packet number presence flag (nummber_of_packets_flag), a PTM presence flag (PTM_flag), and a subsidiary audio attribute presence flag (SA_attribute_flag).

When down load size_flag = 1, a download size (down_load_size) exists. When number_of_packets_flag = 1, a download packet number (number_of_packets) exists. When PTM_flag = 1, a subsidiary audio output start time (SA_S_PTM) and a subsidiary video display end time (SA_E_PTM) exist. When SA_attribute_flag = 1, subsidiary audio attribute information (SA_attribute) exsists. In the case where these flags are 0, a corresponding region does not exist.

The download size (down_load_size) describes size of all the subsidiary audio data to be downloaded. The number of download packets (number_of_packets) describes a packet number of data for transferring all of these items of subsidiary audio data. The subsidiary audio output start time (SA_S_PTM) describes an output start time of subsidiary audio data in units of 90 kHz with precision of 33 bits. The subsidiary audio output end time (SA_E_PTM) describes an output end time of subsidiary audio data in units of 90 kHz with precision of 33 bits. The subsidiary audio attribute information (SA attribute) includes: an audio encoding scheme (audio_compression_mode); multi-channel extension information (multichannel extension); an audio type (audio_type); audio application mode (audio_application_mode); quantization number/DRC (quantization/DRC); number of audio channels (number_of_audio_channels); a specific code (specific_code); a specific code extension (specific_code_extension); and application information (application information) or the like.

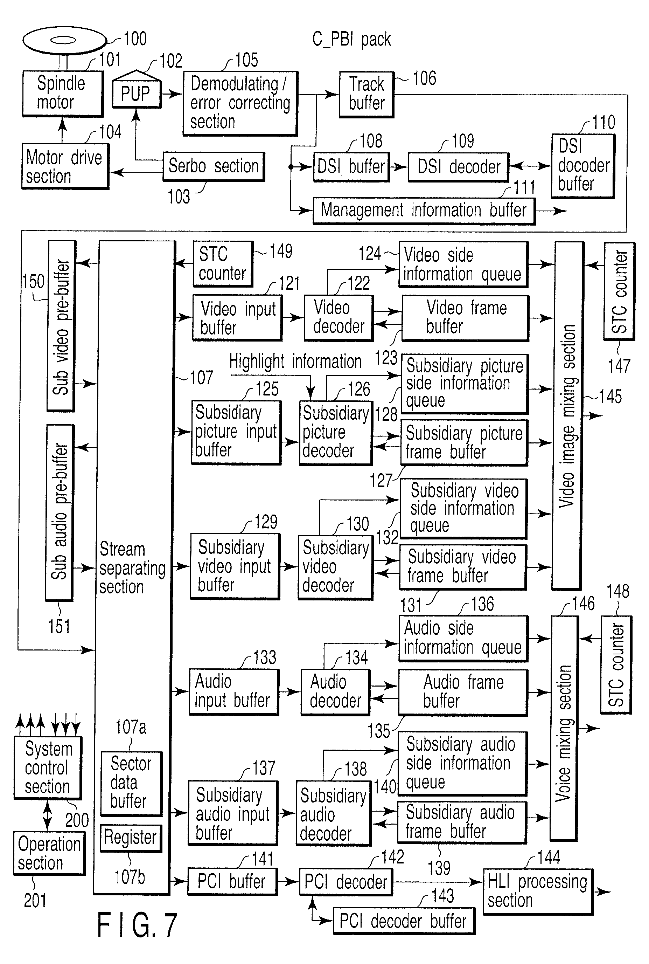

FIG. 7 shows an optical disk device explained in the present embodiment. That is, a recording medium 100 which is an optical disk such as DVD is placed on a turn table (not shown), and is rotationally driven by means of a spindle motor 101.

Assuming that reproduction is currently in progress, the information recorded in the recording medium 100 is read by means of a pickup section 102. The pickup section 102 is subjected to movement control in a disk radial direction, focus control, and tracking control or the like by means of a servo section 103. In addition, the servo section 103 sends a control signal to a motor drive section 104 as well, and makes rotation control of the spindle motor 101 (namely, rotation control of recording medium 100).

An output from the pickup section 102 is inputted to a demodulating/error correcting section 105, and the input is demodulated. Here, the demodulated data is inputted to a stream separating section 107 via a track buffer 106. In addition, the demodulated data is inputted to a DSI decoder 109 via a DSI buffer 108. A DSI decoder buffer 110 is connected to the DSI decoder 109.

The decoded DSI (data search information) is sent to a system control section 200. In addition, the demodulated data is sent to the system control section 200 via a management information buffer 111. VMGI or VTSI and the like is written into this management information buffer 111, and the system control section 200 reads this information and makes reproduction control.

The stream separating section 107 carries out a process for separating packs from each other. First, a description will be given with respect to a processing operation of a video pack (V_PCK), a subsidiary picture pack (SP_PCK), an audio pack (A_PCK), and a navigation pack (NV_PCK).

The data inputted from a track buffer 106 to the stream separating section 107 is captured in a sector data buffer 107a on a one by one sector basis (= by one pack), and type of pack is judged.

A video pack (V_PCK) sampled from the stream separating section 107 is inputted to a video decoder 122 via a video input buffer 121, and the inputted video pack is decoded. A video frame buffer 123 and a video side information queue 124 are connected to the video decoder 122.

Additional information relevant to each of the decoded pictures, for example, a display start time (PTS) or the like is written in the video side information queue 124 by queue. A video image mixing section 145 compares a time of PTS described in the video side information queue 124 with a value of an STC counter 147, and outputs a picture from the video frame buffer 123 for readout display when a display condition is met.

A subsidiary picture pack (SP_PCK) sampled from the stream separating section 107 is inputted to a subsidiary picture decoder 126 via a subsidiary picture input buffer 125, and the inputted pack is decoded. A subsidiary picture frame buffer 127 and a subsidiary picture side information queue 128 are connected to the subsidiary picture decoder 126.

Additional information relevant to each of the decoded subsidiary pictures, for example, a display start time (PTS) or the like is written into the subsidiary picture side information queue 128 by queue. The video image mixing section 145 compares a time of PTS described in the subsidiary picture side information queue 128 with the value of the STC counter 147, and outputs a subsidiary picture from the subsidiary picture frame buffer 127 for readout display when a display condition is met.

An audio pack (A_PCK) sampled from the stream separating section 107 is inputted to an audio decoder 134 via an audio input buffer 133, and the inputted pack is decoded. An audio frame buffer 135 and an audio side information queue 136 are connected to the audio decoder 134.

Additional information relevant to each of the decoded audio frames, for example, an output start time (PTS) or the like is written into the audio side information queue 136 by queue. A voice mixing section 146 compares a time of PTS described in the audio side information queue 136 with a value of an STC counter 148, and reads out and output an audio frame from the audio frame buffer 135 when a display condition is met.

In addition, the stream separating section 107 samples only a PCI packet (PCI_PKT) from a navigation pack (NV_PCK) and discards a DSI packet (DSI_PKT). The sampled PCI packet (PCI_PKT) is inputted to a PCI decoder 142 via a PCI buffer 141, and the inputted packet is decoded.

A PCI decoder buffer 143 is connected to the PCI decoder 142. An output of the PCI decoder 142 is inputted to a highlight information (HLI) processing section 144. In addition, the system control section 200 reads information other than the highlight information (HLI) contained in the PCI data, i.e., PCI general information (PCI_GI), non-seamless angle information (NSML_AGLI), and recording information (RECI) or the like from the PCI decoder buffer 143 via the PCI decoder 142.

Now, a processing operation of the above subsidiary video pack (SV_PCK) 11 and subsidiary audio pack (SA_PCK) 12 will be described here.

When the stream separating section 107 receives a first subsidiary video pack (SV_PCK) 11, additional information (additional_information) which exists in its private data region is sampled in a resistor 107b. In this manner, in a subsidiary video packet (SV_PKT) including the beginning of subsidiary video data, there must exist: a download size (down_load_size); number of download packet (number of_packets); a subsidiary video display start time (SV_S_PTM); a subsidiary video display end time (SV_E_PTM); and subsidiary video attribute information (SV_attribute).

When the stream separating section 107 separates the subsidiary video pack (SV_PCK) 11, subsidiary video data is written into a subsidiary video pre-buffer 150. Then, the stream separating section 107 compares a subsidiary video display start time (SV_S_PTM) with a value of an STC counter 149; reads out subsidiary video data from the subsidiary video pre-buffer 150 when a display condition is met; and inputs the read out data to a subsidiary video decoder 130 via a subsidiary video input buffer 129.

A subsidiary video frame buffer 131 and a subsidiary video side information queue 132 are connected to the subsidiary video decoder 130. Additional information relevant to each of the decoded pictures, for example, a display start time (PTS) or the like is written in the subsidiary video side information queue 132 by queue. The video image mixing section 145 compares a time of PTS described in the subsidiary video side information queue 132 with a value of the STC counter 147, and outputs a picture from the subsidiary video frame buffer 131 for readout display when a display condition is met.

When the stream separating section 107 receives a first subsidiary audio pack (SA_PCK) 12, additional information (additional_information) which exists in its private data region is sampled in the register 107b. In this manner, in a subsidiary audio packet (SA_PKT) 12 including the beginning of subsidiary audio data, there must exist: a download size (down_load_size); number of download packets (number_of_packets); a subsidiary audio output start time (SA_S_PTM); a subsidiary audio output end time (SA_E_PTM); and a subsidiary audio attribute information (SA_attribute).

When the stream separating section 107 separates a subsidiary audio pack (SA_PCK) 12, subsidiary audio data is written into a subsidiary audio pre-buffer 151. Then, the stream separating section 107 compares a subsidiary audio output start time (SA_S_PTM) with a value of the STC counter 149; reads out subsidiary audio data from the subsidiary audio pre-buffer 151 when an output condition is met; and inputs the read out data to a subsidiary audio decoder 138 via a subsidiary audio input buffer 137.

A subsidiary audio frame buffer 139 and a subsidiary audio side information queue 140 are connected to the subsidiary audio decoder 138. Additional information relevant to each of the decoded subsidiary audio frames, for example, a display start time (PTS) or the like is written into the subsidiary audio side information queue 140 by queue. The voice mixing section 146 compares a time of PTS described in the subsidiary audio side information queue 140 with a value of an STC counter 148, and reads out and outputs a subsidiary audio frame from the subsidiary audio frame buffer 139 when an output condition is met.

The system control section 200 disables a user operation control by an operating section 201 based on VOBU user operation control (VOBU_UOP_CTL) and PGC user operation control (PGC_UOP_CTL). In this manner, during stream creation, while subsidiary video data or subsidiary audio data is captured in each of the prebuffers 150 and 151, it is possible to disable a specific reproducing processing operation such as chapter search, title search and I-picture search.

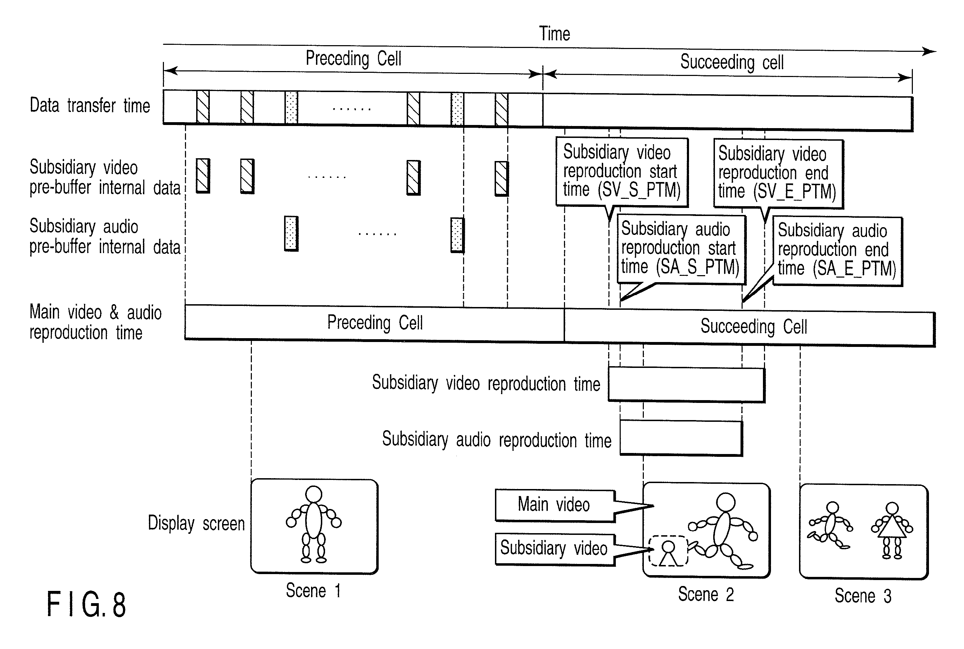

FIG. 8 schematically shows an operation of the above described optical disk device. For example, in preceding cells, subsidiary video data and subsidiary audio data are superimposed as a packet of private_stream_id_2, and a subsidiary video display start time (SV_S_PTM) and a subsidiary audio output start time (SA_S_PTM) are described so as to fall on a reproduction period of the succeeding cells.

In addition, with respect to VOBU included in the preceding cells, it is possible to disable a processing operation such as chapter search, title search and I-picture search by VOBU user operation control (VOBU_UPOP_CTL). By doing this, it becomes possible to reproduce subsidiary video and subsidiary audio in synchronism with main video and audio without being subjected to a limitation that a data retention period in an input buffer of a decoder is equal to or shorter than 1 second, the limitation being defined in an MPEG2 program stream.

According to the above described embodiment, subsidiary video data or subsidiary audio data is padded as private_stream_2 in the MPEG2 program stream which is a main stream of a recording medium 100, thereby making it possible to acquire a subsidiary stream such as subsidiary video data and subsidiary audio data while the main stream is reproduced.

The subsidiary video data and subsidiary audio data are padded as private_stream_2 because these items of data are not subjected to a limitation that the data retention period in the input buffer of the decoder is equal to or shorter than 1 second, the limitation being defined in the MPEG2 program stream. Namely, in the case where subsidiary video data is provided as non-compression data or data having a low compression rate, consideration is taken into the fact that there is a possibility of disabling capturing of one-frame data within one second in the input buffer.

In addition, a reproduction start time (SV_S_PTM, SA_S_PTM) and a reproduction end time (SV_E_PTM, SA_E_PTM) are described in the elementary data of private_stream_2, thereby making it possible to easily obtain synchronization between a main video image and a main voice.

Further, a period including a subsidiary video or subsidiary audio stream has a function of disabling specific reproduction such as user reproduction skip or high speed reproduction, thereby making it possible to guarantee reliable reproduction of subsidiary video or subsidiary audio data.

Further, after a privileged video image/voice has been padded in subsidiary video or subsidiary audio data, specific reproduction such as skip or high speed reproduction is carried out during a period in which these items of data are superimposed, thereby making it possible to notify to a user (by a content provider) in advance by forced display of subtitles (or main video image) that the privileged video image/voice cannot be reproduced. During this period, it is possible to supply CM of company, for example, as a main stream.

The present invention is not limited to the above described embodiment as it is. At the stage of carrying out the invention, the present invention can be embodied by variously modifying constituent elements without departing from the spirit of the invention. In addition, a variety of inventions can be formed by properly combining a plurality of constituent elements disclosed in the above described embodiment. For example, some of the constituent elements may be deleted from all the constituent elements disclosed in the embodiment. For example, the constituent elements according to the different embodiments may be properly combined with each other.

## Claims

1. An information recording medium configured to have, in a region **characterized in that** a main stream is recorded, a region in which at least one of subsidiary video data (11) and subsidiary audio data (12) packed after identification information (stream_id) has been assigned thereto is recorded.

2. An information recording medium according to claim 1, **characterized in that** the main stream is an MPEG2 program stream, and at least one of the video data and audio data is packed as a packet identified by private_stream_2.

3. An information recording medium according to claim 1, **characterized in that** a pack (11, 12) having at least one of the subsidiary video data and subsidiary audio data includes information (SV_S_PTM, SA_S_PTM, SV_E_PTM, SA_E_PTM) indicating a reproduction start time and a reproduction end time of the data.

4. An information recording medium according to claim 1, **characterized in that** the main stream includes at least one of video data (V_PCK) and audio data (A_PCK) packed after identification information (stream_id) has been assigned thereto.

5. An information reproducing apparatus for reproducing an information recording medium (100) having a region in which at least one of subsidiary video data (11) and subsidiary audio data (12) packed after identification information (stream_id) has been assigned thereto is recorded in a region in which a main stream is recorded, the apparatus **characterized by** comprising:
a reproducing section (122, 134) configured to read the main stream from the information recording medium (100) and to apply a decode processing operation to at least one of video data (V_PCK) and audio data (A_PCK) packed after identification information (stream_id) included in the main stream has been assigned thereto; and
a storage section (150, 151) configured to store at least one of the subsidiary video data (11) and subsidiary audio data (12) packed after identification information (stream_id) included in the main stream read from the information recording medium (100) has been assigned thereto during a decode processing operation using the reproducing section (122, 134).

6. An information reproducing apparatus according to claim 5, **characterized by** further comprising an output section (145, 146) configured to output the data decoded by the reproducing section (122, 134) and the data stored in the storage section (150, 151) in synchronism with each other, based on information (SV_S_PTM, SA_S_PTM, SV_E_PTM, SA_E_PTM) indicating a reproduction start time and a reproduction end time of the data, included in a pack (11, 12) having at least one of the subsidiary video data and subsidiary audio data.

7. An information reproducing apparatus according to claim 5, **characterized by** further comprising a control section (200) configured to disable a specific reproducing processing operation during a period in which the storage section (151, 151) stores at least one of the subsidiary video data (11) and subsidiary audio data (12) packed after the identification information (stream_id) has been assigned thereto.

8. An information reproducing method for reproducing an information recording medium (100) which has a region in which at least one of subsidiary video data (11) and subsidiary audio data (12) packed after identification information (stream_id) has been assigned thereto is recorded in a region in which a main stream is recorded, the method **characterized by** comprising:
a reproducing step (122, 134) of applying a decode processing operation to at least one of video data (V_PCK) and audio data (A_PCK) packed after reading the main stream from the information recording medium (100) and identification information (stream_id) included in the main stream has been assigned thereto; and
a storing step (150, 151) of storing at least one of the subsidiary video data (11) and subsidiary audio data (12) packed after identification information (stream_id) included in the main stream read from the information recording medium (100) has been assigned thereto during a decode processing operation by the reproducing step (122, 134).

9. An information reproducing method according to claim 8, **characterized by** further comprising an output step (145, 146) of outputting the data decoded in the reproducing step (122, 134) and the data stored in the storing step (150, 151) in synchronism with each other, based on information (SV_S_PTM, SA_S_PTM, SV_E_PTM, SA_E_PTM) indicating a reproduction start time and a reproduction end time of the data, included in a pack (11, 12) having at least one of the subsidiary video data and subsidiary audio data.

10. An information reproducing method according to claim 8, **characterized by** further comprising a control step (200) of disabling a specific reproducing processing operation during a period in which at least one of the subsidiary video data (11) and subsidiary audio data (12) packed after the identification information (stream id) has been assigned thereto is stored by the storing step (150, 151).
